# EUROPÄISCHE PATENTSCHRIFT

(11) **EP 4 440 646 B1**
(45) Veröffentlichungstag und Bekanntmachung des Hinweises auf die Patenterteilung: **19.03.2025**
(21) Anmeldenummer: 23772798.7
(22) Anmeldetag: 13.09.2023
(51) Int. Cl.: A61M 1/00, A61M 1/36

(54) **BLUTBEUTEL ZUM AUFFANGEN UND WIEDERVERWENDEN VON WUNDBLUT WÄHREND OPERATIONEN**
BLOOD BAG FOR COLLECTING AND RE-USING BLOOD LOST DURING OPERATIONS
POCHE DE SANG POUR COLLECTE ET RÉUTILISATION DU SANG PERDU PENDANT LES OPÉRATIONS

(30) Priorität: 13.10.2022 CH 12092022
(43) Veröffentlichungstag der Anmeldung: 09.10.2024
(73) Patentinhaber: HALILI, Ardian, 5430 Wettingen (CH)
(72) Erfinder: HALILI, Ardian, 5430 Wettingen (CH)
(74) Vertreter: Felber, Josef
(86) Internationale Anmeldenummer: PCT/EP2023/075176
(87) Internationale Veröffentlichungsnummer: WO 2024/078810

(56) Entgegenhaltungen:
- DE-A1- 19 507 580
- DE-U1- 202007 007 136
- US-A- 4 898 572
- US-A1- 2010 130 957

## Beschreibung

Diese Erfindung betrifft einen Blutbehälter, um bei unerwartetem Blutverlust im Zuge einer Operation in einem Operationssaal das Blut des Patienten aufzufangen und nach einer Reinigung mit maschineller Autotransfusion MAT, zum Beispiel in einem sogenannten Cell Saver, gleich wieder verwenden zu können. Zwar sind im Stand der Technik Behälter zur Aufnahme von Wundblut bekannt. So etwa offenbart US 4 898 572 A eine Vorrichtung mit einer Filterkammer und einem integrierten Beutel aus nachgiebigem Kunststoffmaterial zum Sammeln von Patientenblut. Hierzu wird im Beutel ein Unterdruck via einen denselben umgebenden Luftmantel erzeugt. US 2010/130957 A1 indessen handelt von einem stauchbaren Flüssigkeitsbehälter mit einem festen Deckel mit Ein- und Auslässen. Der Flüssigkeitsbehälter kann auch als Saugkanister verwendet werden kann. Trotz solcher bekannter Behälter lässt sich bei auftretenden Blutverlusten während Operationen das patienteneigene Wundblut für seine allfällige Wiederverwendung nach wie vor nicht bedarfsgerecht bereitstellen.

Blutverlust tritt bei allen invasiven Operationen auf. Solches Blut wird bisher zusammen mit dem Natriumchlorid, das für die ständige Wundreinigung eingesetzt wird, in einen Beutel abgesaugt, wozu es Vakuumleitungen bzw. Unterdruckleitungs-Anschlüsse in den Operationssälen gibt. Ein Schlauch führt vom Vakuumanschluss in den Beutel, und ein weiterer Schlauch, ein Drainage-Schlauch mit Ansaugöffnung, führt vom Operationstisch zum Beutel hin und dort in der Nähe des Stutzens für die Vakuumleitung in den Beutel hinein.

Während der Operation, wenn der Patient blutet, wird die Wunde laufend mit Natriumchlorid gewaschen und das austretende Blut wird zusammen mit dem verwendeten Natriumchlorid aufgrund des erzeugten Luftstroms in der Ansaugleitung laufend, ähnlich wie der Speichel beim Zahnarzt, abgesaugt und gelangt über diesen Schlauch in den Auffangbeutel. Nach der Operation wird dieser Blutbeutel bisher durchwegs entsorgt. Das Blut ist verloren.

Nur wenn die Operation erwarten lässt, dass von Anfang an mit einem hohen Blutverlust von mehr als 500 ml zu rechnen ist, wird ein Blutreinigungsgerät in den Operationssaal gestellt, ein sogenannter Cell Saver. Zu diesen Operationen gehören Herz- und Gefässoperationen, abdominal-chirurgische und transplantations-chirurgische Eingriffe, orthopädische und unfallchirurgische Operationen und mit Einschränkungen auch Tumor-Operationen und geburtshilfliche Eingriffe. Die maschinelle Autotransfusion MAT mittels eines Cell Savers hilft grundsätzlich, den Fremdblutbedarf und die damit verbundenen Risiken auf ein Minimum zu senken. Die Kosten pro Einsatz eines Cell Savers liegen zwischen CHF 180.- bis CHF 250.- pro Operation.

Wenn ein Cell Saver eingesetzt wird, so wird das abgesaugte Blut über den Drainage-Schlauch direkt in den Cell Saver geleitet, wo allfällig im Blut vorhandene Feststoffe ausgefiltert werden und das Blut gereinigt wird. Alle auf dem Markt befindlichen Systeme arbeiten nach dem Zentrifugenprinzip. Nach Auffangen des Wund- oder Drainageblutes darf dieses dem Patienten nicht einfach ohne Aufarbeitung zurückgegeben werden. Die Gründe dafür sind unter anderem die Gefahr einer Gerinnungsaktivierung sowie das Risiko einer Überschwemmung des Patienten mit Zytokinen, Endotoxinen und anderen biologisch aktiven Substanzen. Das Blut wird deshalb im Aufarbeitungsgerät filtriert und zentrifugiert. Hierbei werden insbesondere folgende Bestandteile entfernt:
- Aktivierte und nicht aktivierte Gerinnungsfaktoren
- Komplementfaktoren
- Freies Hämoglobin
- Thrombozyten
- Leukozyten (je nach System bis zu 99% Reduktion)
- Heparin
- Antibiotika
- Fett (je nach System bis zu 99.8%)
- Entzündungsmediatoren

Im Gegenzug werden die Erythrozyten angereichert. Das aufbereitete Blut wird über einen Ausgabeschlauch einem hängenden Blutbeutel zugeführt und von diesem aus via Infusion dem Patienten zugeführt. Dieses Blut enthält dann pro 500 ml Volumen 25'000 IE eines Blutgerinnungshemmers, zum Beispiel Heparin oder Natriumcitrat. 25'000 IE reichen dabei für bis zu 3 Liter Blut.

Erleidet ein Patient während einer Operation, bei der man es nicht erwartet, trotzdem einen hohen Blutverlust, so steht regelmässig kein Cell Saver im Operationssaal zur Verfügung. In der Folge wird das Blut in einen herkömmlichen Blut-Aufnahmebeutel abgesaugt, der bis zu 3 Liter fasst. Dieses Blut kann allerdings nicht weiterverwendet werden, da es mit Partikeln verunreinigt sein kann und teilweise auch schon geronnen ist. Dieses Blut ist verloren und muss entsorgt werden und es muss deshalb in einem solchen Fall von unerwartet hohem Blutverlust bisher durchwegs Fremdblut eingesetzt werden, das in der Regel innert ca. 30 Minuten verfügbar ist. Dieses Fremdblut muss aber zuerst geprüft werden, - es muss der richtigen Blutgruppe zugehören und es muss ausserdem geprüft werden, ob der Patient in seinem Blut nicht etwa irgendwelche Antikörper hat, welche dieses Fremdblut für ihn unverträglich machen würden. Wenn die Prüfung erfolgreich ist, kann das Fremdblut über eine Infusion dem Patienten zugeführt werden. Bis es effektiv für eine Infusion zur Verfügung steht, verstreichen ab der Bestellung spitalintern etwa 30 Minuten, und maximal bis zu 3 Stunden. In Fällen, wo versehentlich die Aorta eines Patienten angeschnitten wird, werden Masseninfusionen benötigt, ungefähr 20 bis 40 Standard-Blutbeutel.

Die Problematik des gegenwärtig gebräuchlichen Blutmanagements kann an praktischen Beispielen aufgezeigt werden: Beim routinemässigen Einsetzen von Hüftprothesen wurden in der Praxis Blutverluste von 200 bis 700 ml beobachtet. Der Mensch besitzt pro kg Körpergewicht ungefähr 70 ml Blut. Wenn eine leichtgewichtige Person zum Beispiel 45 kg wiegt, hat sie also ca. 3150 ml Blut in sich, und wenn sie dann 1000 ml Blut verliert, so wird das kritisch. Die Person kann mit so wenig Blut entsprechend weniger Sauerstoff aufnehmen. Die Infektionsgefahr steigt und wenn solches passiert, wird ein Patient einen Tag länger im Spital zurückbehalten, mit entsprechenden Kostenfolgen. Generell lohnt sich eine Blutwäsche bereits ab einem Blutverlust von ca. 500 ml. Viel einfacher und zielführender wäre es, wenn in einem solchen Fall von unerwartet hohem Blutverlust Wundblut verwendet werden könnte. Dafür aber sind die Operationssäle und ihre Ausrüstung bisher nicht gerüstet. Das unerwartet austretende Blut geht leider regelmässig verloren.

Angesichts des geschilderten Sachverhaltes ist es die Aufgabe der vorliegenden Erfindung, im Grundsatz den Einsatz von Fremdblut möglichst entbehrlich zu machen, und dies vor allem in Fällen eines unerwartet hohen Blutverlustes, wenn also kein Blutreinigungs-Gerät bzw. kein Cell Saver im Operationssaal zur Verfügung steht. Vielmehr soll in einem solchen Fall nach der Erfindung das Wundblut trotzdem wiederverwendet werden können.

Diese Aufgabe wird gelöst von einem Blut-Auffangbehälter gemäss den Merkmalen des unabhängigen Anspruchs 1. Bevorzugte Ausführungsformen des Blut-Auffangbehälters sind in den abhängigen Ansprüchen wiedergegeben.

Der Behälter wird als beispielsweise Ausführung in den Figuren dargestellt und im Folgenden beschrieben.

Es zeigt:
- Figur 1:: Einen herkömmlichen Blut-Auffangbehälter in Form einer Flasche;
- Figur 2:: Den Blut-Auffangbehälter nach der Erfindung, steril in einem Lieferbeutel verpackt;
- Figur 3:: Den Blut-Auffangbehälter von oben gesehen;
- Figur 4:: Das untere Ende des ausgepackten Blut-Auffangbehälters mit dem extra angeordneten Ausguss-Stutzen mit Schlauchklemme, zum Abführen von Blut in einen Cell Saver;
- Figur 5:: Den aufgehängten Blut-Auffangbehälter im Einsatz in einem Operationssaal, mit oben einem Zufuhrschlauch für das Drainageblut, einem Luft-Absaugschlauch zu einem Vakuumanschluss und unten dem Abfluss-Schlauch zum Zuführen von Blut in einen Cell Saver.

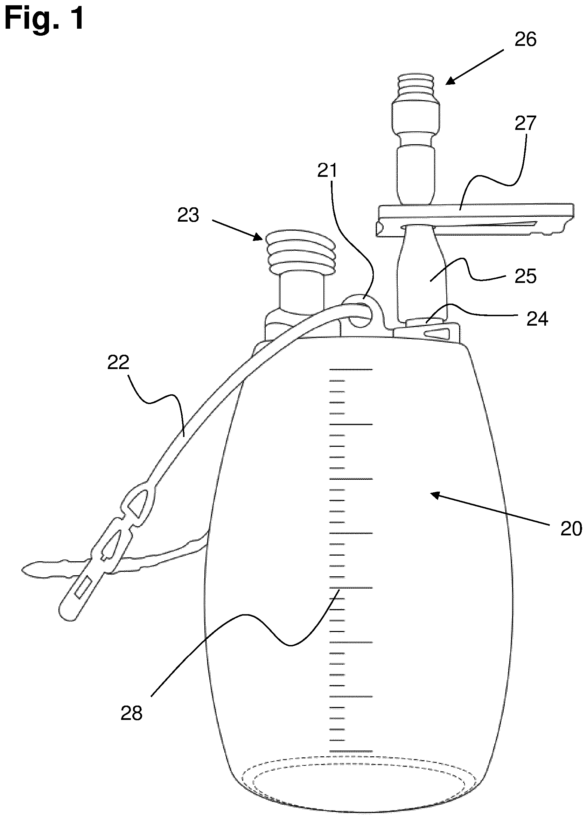

Figur 1 zeigt zunächst einen herkömmlichen Blut-Auffangbehälter in Form einer Auffangflasche 20 aus Kunststoff. Sie weist oben eine Lasche 21 und einen zugehörigen Kunststoff-Binderstreifen 22 auf, sodass sie damit leicht an einem Ständer oder Haken neben dem Operationstisch aufgehängt werden kann. Oben münden zwei Stutzen 23, 24 nach aussen. Der eine Stutzen 23 dient zum Anschluss eines Vakuumschlauches von einem stationären Vakuumanschluss im Operationssaal, der andere Stutzen 24 dient zum Anschluss eines Drainage-Schlauches, der am anderen Ende einen Drainage-Ansaugstutzen aufweist, zum Absaugen von Wundblut aus der blutenden Stellen des Patienten, das heisst wo dieser eine Wunde aufweist oder sein Körper zu Operationszwecken geöffnet wurde. Es ist hier ein Schlauchabschnitt 25 über diesen Stutzen 24 gestülpt, der an seinem hier oberen Ende abermals einen Stutzen 26 bildet, an den der Drainage-Schlauch angeschlossen werden kann. Der Schlauchabschnitt 25 ist mit einer Schlauchklemme 27 ausgerüstet, um beim Wechseln der Flasche - wenn diese voll ist - vorübergehend den Zufluss von Blut zu unterbinden. Die Wunden oder Öffnungen werden laufend mit Natriumchlorid NaCl gereinigt, das dann mit dem Blut vermengt in diese Auffangflasche 20 gelangt. Aussen an dieser Flasche ist im gezeigten Beispiel eine Mess-Skala 28 angebracht, sodass das Volumen des Füllinhaltes jederzeit ablesbar ist. Wenn eine solche Flasche 20 gefüllt ist, wird sie von einer leeren Flasche abgelöst, aber die volle Flasche und insbesondere das darin aufgefangene Wundblut muss entsorgt werden und ist definitiv verloren, obwohl es sich um wertvolles Wundblut handelt. Im Notfall, wenn der Patient wegen grossen Blutverlustes weiteren Blutes bedarf, wird standardgemäss auf Fremdblut zurückgegriffen. Dieses ist in einem Spital innert 30 Minuten bis max. 3 Stunden verfügbar. Es muss aber erst überprüft werden. Die Blutgruppe muss übereinstimmen, und der Patient darf keine Antikörper im Blut haben, welche eine Inkompatibilität mit dem vorgesehenen Fremdblut bewirken könnten.

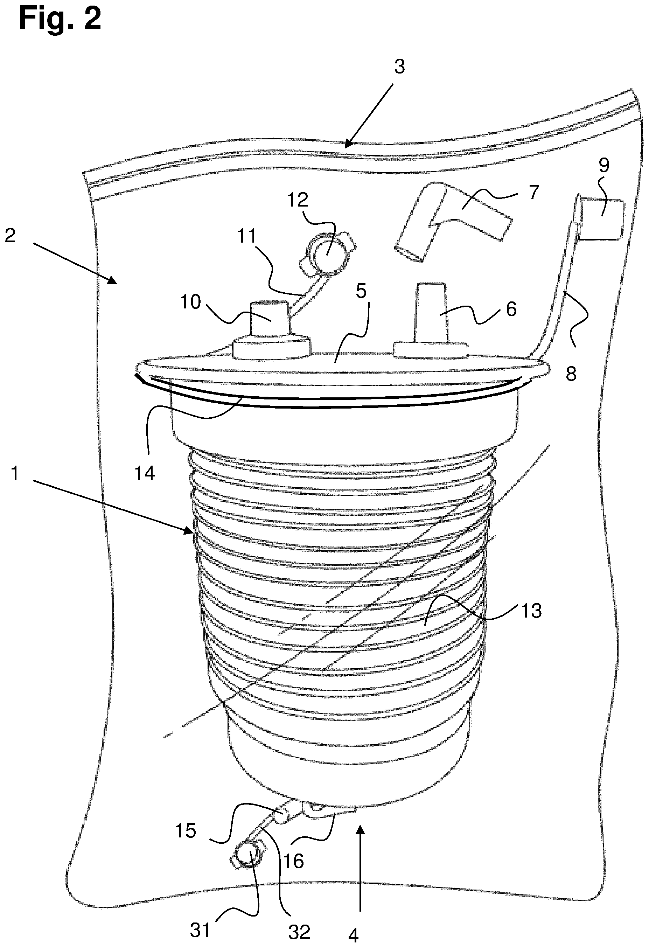

Die Figur 2 zeigt einen Blut-Auffangbehälter 1, hier in Form eines balgartigen Beutels gemäss der vorliegenden Erfindung, hier steril und verschlossen verpackt in einen Plastikbeutel 2 mit oben einem hermetisch verschliessbaren Verschluss 3, also mit einem luft- und wasserdichten Verschluss 3. Als Besonderheit weist dieser Blut-Auffangbeutel 1 an seinem unteren Ende einen weiteren Stutzen 4 zum Anschluss eines Blut-Abführschlauches 30 auf. Hier sieht man ein Stück eines Hilfsschlauches 15, der mit einer Schlauchklemme 16 ausgerüstet ist. Ausserdem gehört zu diesem Hilfsschlauch 15 eine Kappe 31 an einem Sicherungsband 32. Diese Kappe 31 kann mit ihrem Sicherungsband 32 auch am Stutzen 4 angeformt sein. Der Hilfsschlauch 15 oder der anstelle des Hilfsschlauches 15 am Stutzen 4 anzuschliessende Blut-Abführschlauch 30 kann mit dieser Kappe 31 endseitig steril verschlossen werden. Überhaupt kann der Beutel noch einige weitere sterile Reserve-Kappen enthalten, damit er in jedem Fall immer steril verschlossen werden kann, um das Wundblut eines Patienten eine Zeit lang sicher aufbewahren zu können. Auf der Oberseite des Blut-Auffangbehälters 1, hier von einem formstabilen tellerförmigen Deckel 5 gebildet, sind zwei leicht konisch gegen ihr freies Ende hin zulaufende Stutzen 6, 10 angebracht bzw. einstückig mit diesem Deckel 5 in Kunststoff gespritzt. Beidseits des Deckels 5 sind an dessen Rand Aufhängebügel 14 mit ihren beiden Enden einstückig angeformt, wobei diese beiden Aufhängebügel 14 zueinander über den Deckel 5 hochgeschwenkt werden können, wonach der Behälter 1 an diesen beiden Aufhängebügeln 14 mit horizontal liegendem Deckel 5 an einem Haken oder Ständer neben dem Operationstisch aufgehängt werden kann. Der eine Stutzen 6 am Deckel 5 kann wahlweise mit einem Winkelstück 7 ausgerüstet werden, an dessen anderes Ende dann ein Schlauch aufgesteckt werden kann, um über diesen Stutzen 6 Luft abzusaugen, wenn der Schlauch an einen stationären Vakuumanschluss im Operationssaal angeschlossen wird. Das aufgesteckte Winkelstück 7 stellt sicher, dass bei aufgehängtem Behälter der Ansaug-Schlauch, der zum Vakuumanschluss an einer Wand führt, nicht geknickt wird, was sonst den Luftstrom einschränken oder unterbrechen würde. Der Stutzen 6 oder das aufgesetzte Winkelstück 7 kann am freien Ende mit einer Kappe 9 dichtend verschlossen werden. Damit die Kappe 9 nicht verloren gehen kann, ist sie an einem Sicherungsband 8 direkt am Deckel 5 befestigt. Der zweite Stutzen 10 im Deckel 5 dient zum Anschluss eines Drainage-Schlauches, um damit Blut und Natriumchlorid aus Wunden oder geöffneten Körperstellen eines Patienten abzusaugen. Auch dieser Stutzen 10 ist mit einer zugehörigen Kappe 12 an einem Sicherungsband 11 gehalten und ist mit dieser dichtend verschliessbar. Der unten an den Deckel 5 dichtend anschliessende Plastikbeutel des Behälters ist hier als Balg 13 ausgeführt, sodass er mit zunehmender Füllmenge an Blut nach unten wachsen kann, bis er zum Beispiel maximal 3 Liter an Blutvolumen aufnimmt. Als Balg 13 ausgeführt benötigt er im Nichtgebrauch und für Transport- und Lagerzwecke weniger Raum als eine steife Flasche.

Gemäss der Erfindung ist dieser Balg 13 - und das ist sehr wichtig - von allem Anfang an mit einem Mittel zur Hemmung der Blutgerinnung in hinreichender Menge gefüllt. Pro 500 ml Blutvolumen kann das Blut zum Beispiel mindestens 25'000 IE eines Blutgerinnungshemmers namens Heparin enthalten, um die Koagulation des Blutes sicher zu verhindern. 25'000 IE reichen im Fall von Heparin für bis zu 3 Liter Blut. Auch wenn weniger als 500 ml Blut in den Behälter 1 gelangen, sind damit keine Nachteile verbunden, auch wenn er 25'000 IE dieses Blutgerinnungshemmers enthält. Es eignen sich auch alternative Blutgerinnungshemmer, zum Beispiel Natriumcitrat oder andere. Dieser Blut-Auffangbehälter 1 wird also in dem hier in Figur 2 gezeigten Zustand ab Fabrik ausgeliefert und im Spital zwischengelagert, bis man ihn braucht. Seine wichtigen Merkmale sind, dass er erstens an seinem unteren Ende einen Anschluss-Stutzen 4 für den Anschluss eines Blut-Abführschlauches 30 mit einer Schlauchklemme 16 aufweist. Zweitens enthält er ab Fabrik bereits einen Blutgerinnungshemmer in hinreichender Menge für das volle Blutvolumen, das der Behälter 1 aufnehmen kann, zum Beispiel für bis zu 3 Liter Blut. Drittens ist der gesamte Behälter 1, bevor er werkseitig in einen hermetisch verschliessbaren Lieferbeutel 2 verpackt wird, sterilisiert worden. Und er wird unter sterilen Bedingungen in den Lieferbeutel 2 verpackt und in diesem Lieferbeutel 2 im Spital zwischengelagert. Wenn immer der Blut-Aufnahmebehälter 1 zum Einsatz kommt, wird er durch Öffnen des hermetisch verschlossenen Verschlusses 3 aus dem Lieferbeutel 2 entnommen und an einem Ständer im Operationssaal aufgehängt. Dann wird der Blut-Abführschlauch 30 am unteren Stutzen 4 angeschlossen und dieser Schlauch 30 wird entweder mit einer Schlauchklemme 16 verschlossen oder direkt an einen Cell Saver angeschlossen.

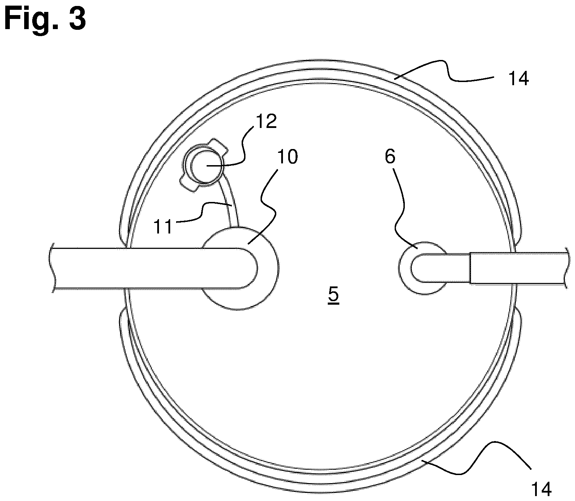

Die Figur 3 zeigt einen Blick von oben auf den Deckel des Blut-Auffangbehälters gesehen. Man erkennt die beiden Stutzen 6, 10 sowie die angeschlossenen Schläuche und eine Kappe 12 mit Sicherungsband 11. Auf beiden Seiten des Umfangs sind einstückig am Deckel 5 angeformte Bügel 14 angeformt, die hier in der Ruhelage gezeigt sind. Sie können bei Bedarf um 90° hochgeschwenkt werden und bilden dann zwei Trägerbügel, an denen der Blut-Aufnahmebehälter an einem Haken aufgehängt werden kann.

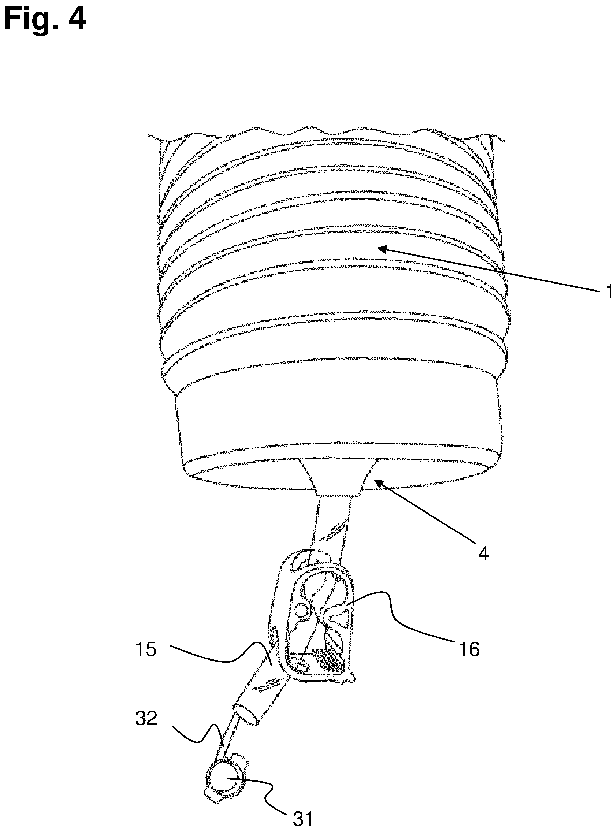

Die Figur 4 zeigt das untere Ende des erfindungsgemässen Blut-Auffangbehälters 1. Hier erkennt man den Abführstutzen 4 sowie einen daran angesteckten Hilfsschlauch 15 mit einer Schlauchklemme 16. Ebenfalls vorhanden ist eine sterile Abdeckkappe 31, die an einem Sicherungsband 32 hängt, damit sie stets zur Verfügung ist, um den Hilfsschlauch 15 oder einen anzuschliessenden Blut-Abführschlauch 30 steril zu verschliessen. Zum Anschliessen des effektiven Blut-Abführschlauches 30 wird dieser kleine Schlauchabschnitt 15 entfernt, und die Schlauchklemme 16 wird auf den effektiven Blut-Abführschlauch aufgeschoben und dann wird die Klemme 16 entweder geschlossen oder für eine geplante direkte Blutabfuhr geöffnet.

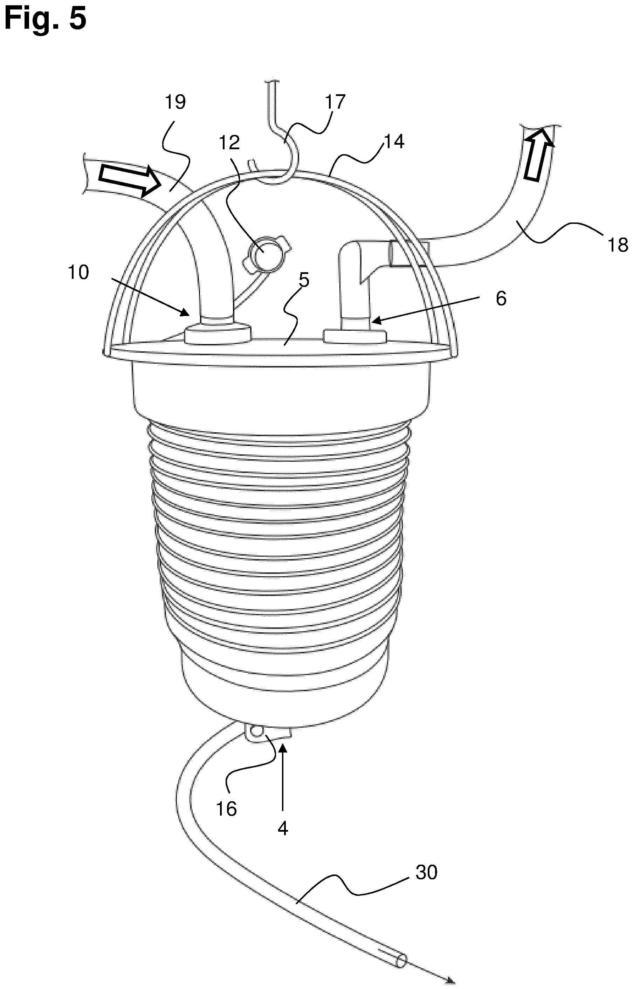

Die Figur 5 zeigt diesen erfindungsgemässen Blut-Auffangbehälter 1 im praktischen Einsatz. Er ist mit seinen beiden Aufhängebügeln 14 an einem Haken 17 aufgehängt. Oben sind an seinem Ansaugstutzen 6 das Winkelstück 7 und ein Absaugschlauch 18 angeschlossen, der zu einem Vakuum-Anschluss führt, wie er in jedem Operationssaal vorhanden ist.

Am anderen Stutzen 10 ist der Blut-Drainage-Schlauch 19 angeschlossen. Unten am Balg 13 des Blut-Auffangbehälters 1, am dortigen Abführstutzen 4 ist der Blut-Abführschlauch 30 angeschlossen, sowie auch die Schlauchklemme 16. Wie im Absaugschlauch 18 und Drainageschlauch 19 mit Pfeilen eingezeichnet, wird Luft über den Absaugschlauch 18 aus dem Behälter 1 abgesaugt, und der induzierte Luftstrom pflanzt sich in den Drainageschlauch 19 fort, sodass mit seinem freien Ende und einem dort aufgesetzten Absaugstück bequem Blut aus Wunden oder geöffneten Patientenstellen abgesaugt werden kann, nach dem gleichen Prinzip wie etwa beim Zahnarzt Speichel aus dem Patientenmund abgesaugt wird.

Wenn die Schlauchklemme 16 im Blut-Abführschlauch 30 verschlossen ist, so wird beim Absaugen laufend Blut in den Behälter 1 gelangen, zusammen mit Natriumchlorid, das für die laufende Wundreinigung eingesetzt wird. Nach und nach wird sich der Behälter 1 mit Blut füllen. Wenn der Behälter 1 voll geworden ist, so können die Schläuche 18, 19 oben entfernt werden und die Stutzen 6, 10 können mit ihren Kappen 9, 12 verschlossen werden. Unten ist der Behälter 1 weiterhin durch die Schlauchklemme 16 dichtend verschlossen. Das aufgefangene Blut des Patienten ist kostbares Wundblut und steht fortan für eine Transfusion zur Verfügung. Es gerinnt nicht und ist steril aufbewahrt. Sollten im Zug einer Operation Komplikationen auftauchen - oder auch nur schon beim Auftreten eines unerwartet hohen Blutverlustes - kann dieses aufgefangene Wundblut dank diesem Behälter 1 direkt transfusioniert werden, sobald ein Cell Saver in den Operationssaal gestellt wird oder dort schon vorhanden ist. Im Fall, dass eine solches Blutreinigungs-Gerät bzw. ein Cell Saver in den Operationssaal gebracht werden muss, muss das selbstverständlich steril erfolgen, also darf nur ein zuvor sterilisiertes Blutgerät in den Operationssaal gebracht werden. Der Blut-Abführschlauch 30 wird für diesen Fall an den unteren Stutzen 4 und mit seinem anderen Ende an den Cell Saver angeschlossen, sodass das aufgefangene Wundblut im Cell Saver gereinigt werden kann und hernach direkt in einen Infusionsbeutel geleitet werden kann und ab diesem dem Patienten via eine Infusion zugeführt werden kann. Ein Einsatz von Fremdblut und die damit zusammenhängenden logistischen und medizinischen Massnahmen können unterbleiben. Das spart Zeit und Kosten und Infektionen werden vermieden. Ausserdem können Patienten, die relative hohe Blutverluste erlitten haben, früher, meist einen oder gar mehrere Tage früher als sonst, aus dem Spital entlassen werden. Das spart wiederum erhebliche Kosten ein.

Wundblut ist ohnehin das für den Patienten problemloseste Blut und dank diesem Blut-Auffangbehälter 1 geht es nicht mehr wie bisher verloren, sondern steht sofort oder auch mit zeitlichen Unterbrüchen von mehreren Stunden und Tagen dem Patienten jederzeit zur Verfügung. Es braucht damit auch niemals mehr ein Patient auf dem Operationstisch zu verbluten.

## Patentansprüche

1. Blut-Auffangbehälter (1) mit oben einem Ansaugstutzen (10) zum Anschluss eines Drainage-Schlauches (19) sowie einem Absaugstutzen (6) zum Anschluss des Saugschlauches (18) eines Vakuum-Anschlusses, wobei der Blut-Auffangbehälter (1) unten einen Abführstutzen (4) zum Abführen von gesammeltem Wundblut über einen Blut-Abführschlauch (30) an ein Blutreinigungsgerät aufweist, und weiter dass der Blut-Auffangbehälter (1) für Wundblut ein Mittel zur Hemmung der Blutgerinnung in hinreichender Menge für ein volles Blutvolumen, das der Behälter (1) aufnehmen kann, enthält und insgesamt sterilisiert ist und in einen mittels hermetischem Verschluss (3) steril verschlossenen Lieferbeutel (2) verpackt ist, zur Öffnung im Operationssaal kurz vor Gebrauch.

2. Blut-Auffangbehälter (1) nach Anspruch 1, wobei am unteren Abführstutzen (4) ein Schlauchabschnitt (15) als Hilfsschlauch aufgesetzt ist, zum Anschliessen des Blut-Abführschlauches (30) an diesen Abfuhrstutzen (4), wobei der Schlauchabschnitt (15) mit einer Schlauchklemme (16) verschliessbar ist, sodass mittels der Schlauchklemme (16) der Schlauchabschnitt (15) bzw. der anzuschliessende Blut-Abführschlauch (30) wahlweise geöffnet oder verschlossen werden kann, und wobei am Abführstutzen (4) oder am Schlauchabschnitt (15) eine Verschlusskappe (31) über ein Sicherungsband (32) angeformt ist.

3. Blut-Auffangbehälter (1) nach Anspruch 1, wobei der Behälter (1) als Balg (13) ausgeführt ist, sodass im hängenden Zustand sein Volumen mit der Menge des zugeführten Blutes nach unten ausdehnbar ist und er nach unten zu seinem vollen Volumen wachsen kann.

4. Blut-Auffangbehälter (1) nach einem der vorangehenden Ansprüche, wobei der Behälter (1) oben mit einem tellerförmigen Deckel (5) dichtend abgeschlossen ist, wobei der Deckel (5) von den zwei Stutzen (6,10)zum Anschluss des Absaugschlauches (18) bzw. des Drainageschlauches (19) durchbrochen ist.

5. Blut-Auffangbehälter (1) nach Anspruch 4, wobei die Stutzen (6, 10) von je einer Kappe (9, 12), die über je ein Sicherungsband (8, 11) am Deckel (5) gehalten sind, dichtend verschliessbar sind.

6. Blut-Auffangbehälter (1) nach einem der vorangehenden Ansprüche, wobei auf den Stutzen (10) ein Winkelstück (7) aufsetzbar ist, zum Vermeiden von Knickstellen im ab- oder zuführenden Vakuum- (18) oder Drainage-Schlauch (19).

7. Blut-Auffangbehälter (1) nach einem der Ansprüche 4 bis 6, wobei er an seinem oberen Deckel angeformt zwei Aufhängebügel (14) aufweist, die zueinander hochschwenkbar sind, zum Aufhängen des Blut-Auffangbehälters (1) an einem Haken.

8. Blut-Auffangbehälter (1) nach einem der vorangehenden Ansprüche, wobei das Blutvolumen, das der Behälter (1) aufnehmen kann, 3 Litern Blut entspricht.

## Claims

1. Blood collection container (1) with a suction nozzle (10) at the top for connecting a drainage hose (19) and a suction nozzle (6) for connecting the suction hose (18) of a vacuum connection, wherein the blood collection container (1) has a discharge nozzle (4) at the bottom for discharging collected wound blood via a blood discharge hose (30) to a blood purification device, and further in that the blood collection container (1) for wound blood contains an agent for inhibiting blood coagulation in sufficient quantity for a full volume of blood which the container (1) can hold, and is sterilised as a whole and is packed in a delivery bag (2) which is sterilely sealed by means of a hermetic closure (3), for opening in the operating theatre shortly before use.

2. Blood collection container (1) according to claim 1, wherein a hose section (15) is fitted to the lower discharge nozzle (4) as an auxiliary hose for connecting the blood discharge hose (30) to this discharge nozzle (4), wherein the hose section (15) can be closed with a hose clamp (16), so that by means of the hose clamp (16) the hose section (15) or the blood discharge hose (30) to be connected can be selectively opened or closed, and wherein a closure cap (31) can be fitted to the discharge nozzle (4) or to the hose section (15) via a securing means. the blood discharge hose (30) to be connected can be optionally opened or closed by means of the hose clamp (16), and wherein a sealing cap (31) is moulded onto the discharge nozzle (4) or the hose section (15) via a securing band (32).

3. Blood collection container (1) according to claim 1, wherein the container (1) is designed as a bellows (13), so that in the suspended state its volume can expand downwards with the amount of blood supplied and it can grow downwards to its full volume.

4. Blood collection container (1) according to one of the preceding claims, wherein the container (1) is sealed at the top with a disc-shaped lid (5), wherein the lid (5) is perforated by the two nozzles (6, 10) for connecting the suction hose (18) or the drainage hose (19).

5. Blood collection container (1) according to claim 4, wherein the nozzles (6, 10) can each be sealed by a cap (9, 12), which are each held on the lid (5) by a securing band (8, 11).

6. Blood collection container (1) according to one of the preceding claims, wherein an angle piece (7) can be placed on the connecting piece (10) to avoid kinks in the outgoing or incoming vacuum (18) or drainage hose (19).

7. Blood collection container (1) according to one of claims 4 to 6, wherein it has two suspension brackets (14) moulded onto its upper lid, which can be swivelled up towards each other, for suspending the blood collection container (1) from a hook.

8. Blood collection container (1) according to one of the preceding claims, wherein the volume of blood that the container (1) can hold corresponds to 3 litres of blood.

## Revendications

1. Récipient de collecte de sang (1) avec en haut une tubulure d'aspiration (10) pour le raccordement d'un tuyau de drainage (19) ainsi qu'une tubulure d'aspiration (6) pour le raccordement du tuyau d'aspiration (18) d'un raccord de vide, le récipient de collecte de sang (1) présentant en bas une tubulure d'évacuation (4) pour l'évacuation du sang de plaie collecté par un tuyau d'évacuation de sang (30) vers un appareil de purification de sang, et en ce que le récipient de collecte de sang (1) pour le sang de la plaie contient un agent inhibiteur de la coagulation du sang en quantité suffisante pour un volume entier de sang que le récipient (1) peut contenir, et est stérilisé dans son ensemble et emballé dans un sac de livraison (2) fermé de manière stérile au moyen d'une fermeture hermétique (3), pour être ouvert dans la salle d'opération juste avant l'utilisation.

2. Récipient collecteur de sang (1) selon la revendication 1, dans lequel un tronçon de tuyau (15) est placé sur la tubulure d'évacuation inférieure (4) en tant que tuyau auxiliaire, pour le raccordement du tuyau d'évacuation de sang (30) à cette tubulure d'évacuation (4), dans lequel le tronçon de tuyau (15) peut être fermé par un collier de serrage (16), de sorte qu'au moyen du collier de serrage (16), le tronçon de tuyau (15) ou le tuyau d'évacuation de sang (30) peut être ouvert ou fermé de manière amovible. le tuyau d'évacuation du sang (30) à raccorder peut être ouvert ou fermé au choix, et dans lequel un capuchon de fermeture (31) est formé sur la tubulure d'évacuation (4) ou sur le tronçon de tuyau (15) par l'intermédiaire d'une bande de sécurité (32).

3. Récipient de collecte de sang (1) selon la revendication 1, dans lequel le récipient (1) est réalisé sous la forme d'un soufflet (13), de sorte qu'à l'état suspendu, son volume est extensible vers le bas avec la quantité de sang amenée et qu'il peut cro tre vers le bas jusqu'à son plein volume.

4. Récipient de collecte de sang (1) selon l'une des revendications précédentes, le récipient (1) étant fermé de manière étanche en haut par un couvercle (5) en forme d'assiette, le couvercle (5) étant traversé par les deux tubulures (6, 10) pour le raccordement du tuyau d'aspiration (18) ou du tuyau de drainage (19).

5. Récipient de collecte de sang (1) selon la revendication 4, dans lequel les tubulures (6, 10) peuvent être fermées de manière étanche par un capuchon respectif (9, 12) qui est maintenu sur le couvercle (5) par une bande de sécurité respective (8, 11).

6. Récipient de collecte de sang (1) selon l'une des revendications précédentes, dans lequel une pièce coudée (7) peut être placée sur la tubulure (10) pour éviter les points de pliage dans le tuyau de vide (18) ou de drainage (19) d'évacuation ou d'alimentation.

7. Récipient de collecte de sang (1) selon l'une des revendications 4 à 6, dans lequel il comporte, formés sur son couvercle supérieur, deux étriers de suspension (14) qui peuvent pivoter vers le haut l'un par rapport à l'autre, pour suspendre le récipient de collecte de sang (1) à un crochet.

8. Récipient de collecte de sang (1) selon l'une quelconque des revendications précédentes, dans lequel le volume de sang que le récipient (1) peut contenir correspond à 3 litres de sang.
